# EUROPEAN PATENT APPLICATION

(11) **EP 4 831 999 A1**
(43) Date of publication of application: **28.10.2026**
(21) Application number: 25172283.1
(22) Date of filing: 24.04.2025
(51) Int. Cl.: G16H 20/30, G16H 40/63, G16H 50/50

(54) **METHOD AND SYSTEM FOR ULTRASOUND NEUROMODULATION STIMULATION GUIDED BY A DOMAIN-SPECIFIC LARGE VISION MODEL**

(71) Applicant: Sanmai Technologies, PBC, Sunnyvale, CA 94087 (US)
(72) Inventor: DAFT, CHRISTOPHER, SUNNYVALE, CA, 94087 (US)
(74) Representative: Araujo, Daniel

(57) **Abstract**

A system (20) for guiding an ultrasound stimulation procedure. The system comprises an ultrasonic probe (22) configured to acquire real-time ultrasound data (23) from a region of interest, an input interface (24) configured to receive a user-specified command indicating a target anatomical structure, and a computing device comprising a trained domain-specific large vision model (DSLVM) (14). The DSLVM (14) is configured to receive the real-time ultrasound data (23) and the user-specified command, and to output anatomical target coordinates (25). A controller (26) computes beamforming parameters based on the target coordinates, and a neuromodulation beamformer (27) delivers focused ultrasound stimulation accordingly. The invention also relates to a computer-implemented method for training the DSLVM (14) using synthetic ultrasound data generated from volumetric anatomical scans (11) and simulated probe specifications (13b).

## Description

### TECHNICAL FIELD

The present invention relates to the field of medical ultrasound systems. In particular, it concerns a system and method for guiding an ultrasound-based neuromodulation procedure using a domain-specific vision model trained on anatomical data.

### PRIOR ART

In medical ultrasound systems, neuromodulation procedures involve the application of focused ultrasound energy to specific anatomical regions of a subject, such as structures within the brain, to influence neural activity. Conventional systems typically rely on a combination of hardware-based beamforming and manual targeting, often requiring an operator (e.g. a clinician) to interpret real-time imaging data or pre-procedure imaging studies to identify and localize the target anatomical structure.

Some systems make use of image processing or conventional computer vision techniques to assist the clinician in identifying anatomical landmarks. However, these techniques often lack robustness and adaptability, particularly in the presence of anatomical variability across subjects or when using low-resolution or slice-based ultrasound data. Moreover, manual targeting can be time-consuming, operator-dependent, and prone to variability.

The accuracy and effectiveness of neuromodulation depend on the precise localization of the target structure, as well as the ability to adjust stimulation timing relative to physiological events. There remains a need for improved targeting and control mechanisms in ultrasound neuromodulation systems, particularly those capable of adapting to real-time imaging input and subject-specific anatomical information.

### SUMMARY OF THE INVENTION

A first aspect of the invention refers to a computer-implemented method for training a domain-specific large vision model (DSLVM) for guiding an ultrasound stimulation system to target specific anatomical locations. The method comprises providing volumetric anatomical data that includes volumetric anatomical scans of target locations representing anatomical structures (e.g. of one or more subjects). These anatomical structures preferably correspond to cranial and/or cerebral regions of the one or more subjects.

The method further comprises providing a specification of an ultrasonic probe of an ultrasound stimulation system, said specification comprising a set of technical parameters defining the functional and hardware configuration of the probe. The technical parameters may comprise one or more of: transducer type (e.g. 1D ultrasonic probe, 2D ultrasonic or 1.75D array), the geometry and spatial arrangement of transducer elements (e.g. elevation and azimuth dimensions), the acoustic characteristics (e.g. centre frequency), and the spatial position and orientation of the probe relative to a target anatomical location). Based on this probe specification and the volumetric anatomical data, synthetic ultrasound training data is generated by simulating (e.g. with a computing device) an ultrasound field produced by the specified ultrasonic probe at multiple probe positions relative to the anatomical structures represented in the volumetric anatomical scans.

The DSLVM is then trained by inputting the synthetic ultrasound training data into the DSLVM. As a result of the training, model parameters are generated that, when used by the DSLVM (e.g. when the DSLVM is integrated into a computing device), enable the DSLVM to identify anatomical target locations from real-time ultrasound data acquired by the specified ultrasonic probe during operation of the ultrasound stimulation system.

In some embodiments, the DSLVM may be trained on a computing platform comprising one or more GPUs and/or CPUs, or a distributed network thereof. The use of GPU-accelerated training infrastructure may reduce model convergence time and support large-scale simulation-based datasets. The computing device may comprise memory storing the DSLVM, its trained model parameters, and/or the synthetic training data.

The term "domain-specific large vision model" or DSLVM refers to a computer-implemented model trained on image-based datasets relating to a particular anatomical domain (e.g. cranial or cerebral structures) for the purpose of performing medical image analysis tasks. The DSLVM may be based on architectures such as transformer-based models, convolutional neural networks (CNNs), or multi-layer perceptrons (MLPs), and may be trained using real or simulated image data. The DSLVM is configured to process ultrasound image data and output anatomical features, coordinates, or derived guidance information suitable for use in an ultrasound stimulation system.

The expression "synthetic ultrasound training data" may be interpreted as referring to simulated ultrasound data generated by modelling the acoustic response of an ultrasonic probe when placed at different positions relative to anatomical structures represented in volumetric anatomical scans. The generation of this data may involve estimating acoustic property maps (e.g. density or speed of sound) and simulating ultrasound propagation using models such as pseudo spectral, finite-element, or finite-difference models.

In the context of the present invention, the term "volumetric anatomical data" (or "volume anatomical data") refers to three-dimensional image data representing anatomical structures of a subject, such as that acquired through magnetic resonance imaging (MRI), computed tomography (CT), or other imaging modalities capable of producing volumetric scans. Such data typically comprises a stack of two-dimensional image slices or a voxel-based image volume, which collectively represent the spatial distribution of anatomical features within a region of interest. Volumetric anatomical data may be used for training the DSLVM, for defining anatomical targets, or for comparison and registration with real-time ultrasound data during system operation.

According to some embodiments, the training of the DSLVM may involve applying a training loop in which predicted anatomical target locations are generated by the DSLVM based on the synthetic ultrasound training data. The training loop may further comprise comparing the predicted locations with target anatomical locations derived from the volumetric anatomical data (e.g. with reference target anatomical locations of the volumetric anatomical data). Based on this comparison, training errors may be computed (e.g. the training error may be interpreted as an output resulting from the comparison), and training feedback may be provided to the DSLVM. The training loop may further comprise iteratively adjusting the model parameters of the DSLVM by providing a training feedback to the DSLVM based on training errors computed from the comparison.

In some embodiments, the reference anatomical target locations used in the training loop may be derived from annotations or labels of target anatomical structures in the volumetric anatomical data. These labels may indicate the presence or boundaries of specific anatomical regions and may be used to compute training errors during the training loop. The training feedback provided to the DSLVM may be based on the training errors computed from the comparison between the predicted anatomical target locations and the reference target locations derived from the labels.

The method may further comprise providing (e.g. selectively providing) a prompt string indicating a specific target anatomical structure during training. The prompt string may serve to condition the DSLVM so that the model associates the ultrasound data with anatomical target locations corresponding to the specific target anatomical structure indicated in the prompt string. The synthetic ultrasound training data may be generated specifically for the target anatomical structure indicated by the prompt string. The term "prompt string" refers to a user-provided input, such as a textual description or label, which is used during the training of the DSLVM to specify an anatomical target of interest. The prompt string may influence the DSLVM's outputs by conditioning the model to identify or focus on a structure corresponding to the indicated anatomical region.

Generating the synthetic ultrasound training data may comprise generating acoustic property maps by processing at least one of the volumetric anatomical scans from the volumetric anatomical data. The acoustic property maps may represent spatial distributions of acoustic parameters of anatomical structures, such as tissue density or speed of sound, and may be used as input to the ultrasound simulation. These maps enable the simulation to produce training data that realistically reflects the acoustic environment of the relevant anatomical region.

In some embodiments, simulating the ultrasound field in order to generate the synthetic ultrasound training data may comprise modelling ultrasound propagation and scattering using a simulation model. The simulation model may comprise one of a pseudo spectral model, a finite-element model, or a finite-difference model. These models may be used to approximate the physical behavior of ultrasound waves as they travel through and interact with anatomical structures (e.g. as those represented in the acoustic property maps), thereby enabling the generation of realistic synthetic ultrasound data for training the DSLVM.

Therefore, the computer-implemented training method of the first aspect of the invention is aimed at training a DSLVM to comprehend the relationship between ultrasound slice or volume data captured by an ultrasonic probe and volume anatomical data from a database.

Although the first aspect of the invention has been described above in terms of a computer-implemented method for training a domain-specific large vision model, it will be understood that the invention may also be formulated in terms of a training system (e.g. configured to carry out the computer-implemented method). Such a system may comprise at least one computing device configured to perform the steps of the method. In some embodiments, the at least one computing device may comprise or be operatively associated with a database (e.g. a memory) for storing volumetric anatomical data, and may further comprise an application configured to execute a simulation of an ultrasound field produced by a specified ultrasonic probe at different positions relative to anatomical structures represented in the volumetric anatomical data. The same application (or an additional application) may be configured to train the DSLVM by inputting the simulated ultrasound training data and iteratively adjusting model parameters based on comparisons between predicted anatomical target locations and reference anatomical target locations to calculate training errors. The trained model parameters may enable the DSLVM to identify anatomical target locations from real-time ultrasound data acquired by the specified ultrasonic probe during operation of the ultrasound stimulation system.

The method and system described above may provide several technical advantages. By simulating ultrasound fields based on subject-specific anatomical data and detailed probe specifications, the DSLVM may be trained with data that realistically reflects the acoustic conditions encountered during operation. This may improve the model's ability to identify target anatomical locations under varying real-time imaging conditions. Furthermore, the integration of prompt-based conditioning, acoustic property modelling, and simulation-based data generation may reduce the reliance on large annotated datasets or manual targeting procedures, thereby enabling more robust, accurate, and operator-independent guidance for ultrasound neuromodulation.

A second aspect of the invention refers to a system for guiding an ultrasound stimulation procedure (the system may also be referred to as a system for guided ultrasound stimulation). The system comprises: an ultrasonic probe, an input interface, a computing device, a controller, and a neuromodulation beamformer.

The ultrasonic probe is configured to acquire real-time ultrasound data from a region of interest within a subject, the region of interest preferably including cranial and/or cerebral anatomical structures. In some embodiments, the system may be configured for use in transcranial focused ultrasound stimulation (tFUS) procedures, wherein focused ultrasound energy is delivered non-invasively through the skull to specific neural targets. The system may be adapted for therapeutic or wellness purposes, such as neuromodulation of cortical or subcortical structures. Depending on the embodiment, the ultrasonic probe may be configured as a 1D ultrasonic probe, as a 2D ultrasonic probe or as a 1.75D ultrasonic probe.

The input interface is configured to receive a user-specified command indicating a target anatomical structure to be targeted. Preferably, the input interface is configured to receive the user-specified command either as a textual input and/or as a set of target points (e.g. a set of target points marked on a pre-procedure auxiliary volumetric anatomical scan provided as an input to the system).

The term "region of interest within a subject" refers to a specific anatomical area of the subject's body that is selected for imaging, targeting, or treatment using the ultrasound stimulation system. The region of interest may include internal anatomical structures such as tissues, organs, or neural regions that are to be visualized or stimulated. In some embodiments, the region of interest preferably comprises cranial and/or cerebral anatomical structures. The region of interest may be defined spatially based on prior imaging data, user input, or the positioning of the ultrasonic probe. The region of interest, and the associated target anatomical structures or locations, may relate to areas intended to be treated either for therapeutic purposes, such as modulation of neurological conditions, or for wellness purposes, such as cognitive enhancement or stress reduction.

The computing device comprises a trained domain-specific large vision model, DSLVM. The DSLVM is configured to receive the real-time ultrasound data and the user-specified command, and to output anatomical target coordinates of the indicated target anatomical structure based on the received data and command. The DSLVM is preferably a model trained using the computer-implemented method according to the first aspect of the invention.

The controller is configured to compute beamforming parameters based on the anatomical target coordinates. The neuromodulation beamformer is coupled to the ultrasonic probe and is configured to generate and deliver focused ultrasound stimulation to the anatomical target locations based on the beamforming parameters.

This configuration may reduce the need for manual interpretation of real-time ultrasound images, and may enable more consistent and operator-independent localization of target anatomical structures during neuromodulation procedures.

According to some embodiments, the DSLVM may be further configured to generate confirmatory outputs comprising a visual representation of anatomical structures and a treatment region corresponding to the anatomical target coordinates. The system may further comprise an output interface configured to provide the confirmatory outputs to an operator (e.g. to a clinician) to facilitate verification of the targeting prior to or during the delivery of ultrasound stimulation (e.g. to a target anatomical location). Preferably, the output interface may be further configured to display a graphical indicator representing a confidence level determined by the DSLVM, based on the user-specified command and the real-time ultrasound data acquired by the ultrasonic probe. The confidence level may indicate the suitability of the current spatial location of the ultrasonic probe for delivering ultrasound stimulation to the target anatomical location specified by the user.

The confirmatory visual representation of anatomical structures and the treatment region may assist the operator in verifying the correctness of targeting prior to or during the procedure. Displaying a graphical indicator of the system's confidence level may further enhance procedural safety and user trust, by providing an intuitive assessment of the suitability of the current probe positioning for delivering stimulation to the intended target.

The system may further comprise one or more physiological sensors configured to monitor a physiological activity of the subject in real time during ultrasound stimulation at the target anatomical location. The physiological sensors preferably comprise electroencephalographic (EEG) or magnetoencephalographic (MEG) sensors. The DSLVM may be further configured to receive the physiological activity measured by the one or more physiological sensors and to generate timing control signals based on the measured physiological activity. The neuromodulation beamformer may be further configured to use the timing control signals to synchronize the delivery of ultrasound stimulation with the physiological activity of the subject.

The integration of real-time physiological sensing may allow for dynamic adjustment of the stimulation timing based on the subject's physiological state. This may improve treatment efficacy and reduce the risk of stimulation being delivered at suboptimal times, thereby enhancing the therapeutic potential of the system.

According to some embodiments, the ultrasonic probe may comprise transducer elements (e.g. a plurality; e.g. a matrix array of transducer elements) configured to acquire volume ultrasound data from the region of interest within the subject. The system may further comprise an output interface configured to display a real-time visualization of the volume ultrasound data acquired by the ultrasonic probe. This configuration may facilitate live anatomical interpretation and enhance the operator's ability to monitor probe positioning and targeting accuracy during the neuromodulation procedure. Preferably, the ultrasonic probe may be configured as a 2D ultrasonic probe. A 2D (two-dimensional) ultrasonic probe features a matrix of transducer elements arranged in a grid pattern, covering both azimuthal and elevation directions. This configuration allows for electronic steering and focusing of the ultrasound beam in both dimensions, enabling direct acquisition of volumetric (3D) data and faster imaging of moving structures.

In some alternative embodiments, the ultrasonic probe may comprise a matrix array of transducer elements configured to acquire slice ultrasound data (i.e. instead of to acquire volume ultrasound data) from the region of interest within the subject. Preferably, the ultrasonic probe may be configured as a 1D or as a 1.75D ultrasonic probe.

A 1D (one-dimensional) ultrasonic probe comprises of a single row of transducer elements (e.g. piezoelectric transducer elements) arranged linearly along one axis (e.g. an azimuthal direction). These elements can be electronically controlled to steer and focus the ultrasound beam within a single imaging plane, allowing for the creation of 2D images (e.g. slice data) by sweeping the beam across the tissue. Types of 1D probes include linear, convex, and phased arrays. A 1.75D ultrasonic probe is an advanced array that bridges the gap between 1D and full 2D arrays. It consists of multiple rows of transducer elements (more than 1D, but not a full 2D matrix), allowing for partial electronic control in both the azimuthal and elevation directions. This design improves image quality by enhancing focus and reducing artifacts in the elevation plane, resulting in better contrast and speckle reduction compared to 1D arrays. The 1.75D array offers many benefits of 2D arrays, such as improved slice thickness control, but with reduced complexity and cost.

The system may further comprise a probe orientation sensor configured to determine an orientation of the ultrasonic probe. The probe orientation sensor may be configured as a MEMS gyro (Micro-Electro-Mechanical Systems gyroscope) or as an accelerometer, preferably attached to the ultrasonic probe. The DSLVM may be further configured to receive orientation data from the probe orientation sensor for use in determining the target anatomical coordinates. The availability of real-time orientation data may improve the spatial contextualization of acquired ultrasound slices, enabling more accurate localization of the target anatomical location even in the absence of volumetric imaging.

The DSLVM may be further configured to receive, via the input interface, a pre-procedure auxiliary volumetric anatomical scan of the subject as input. The pre-procedure auxiliary volumetric anatomical scan may provide subject-specific anatomical information for use by the DSLVM in determining the target anatomical coordinates from the slice ultrasound data acquired by the ultrasonic probe. The use of pre-procedure volumetric anatomical data may enhance anatomical context and spatial registration during targeting, particularly in embodiments employing slice-based ultrasound acquisition.

In some embodiments, the DSLVM may be further configured to evaluate a degree of completeness of the slice ultrasound data acquired by the ultrasonic probe from the region of interest. This evaluation may be performed by comparing the acquired slice ultrasound data with the pre-procedure auxiliary volumetric anatomical scan. Based on this comparison, the DSLVM may generate a completeness output representative of the degree of completeness of the acquired data. The DSLVM may also be configured to determine a recommended position of the ultrasonic probe for completing the acquisition of slice ultrasound data, by comparing the current spatial position of the probe (determined based on the real-time ultrasound data acquired by the ultrasound probe, preferably in combination with orientation data provided by the optional probe orientation sensor) with the pre-procedure scan. A positioning output may be generated, indicating the next recommended probe position. These outputs may assist the operator in navigating the probe to ensure adequate coverage of the region of interest and data quality for accurate targeting.

The DSLVM may be further configured to generate a real-time anatomical model based on a coordinate transformation of the pre-procedure auxiliary volumetric anatomical scan, the real-time ultrasound data acquired by the ultrasonic probe, and the user-specified command indicating the target anatomical structure. The real-time anatomical model is preferably overlaid with the target anatomical coordinates corresponding to the target anatomical location. This real-time model may assist the operator in visualizing the anatomical context of the stimulation site, thereby improving spatial orientation and confidence in targeting precision during the procedure.

According to some embodiments, the array of transducer elements of the ultrasonic probe configured to acquire slice ultrasound data may extend along a first direction and along a second direction, wherein each transducer element has a first dimension extending along the first direction and a second dimension extending along the second direction. The first dimension may be larger than the second dimension. The array may be configured to acquire slice ultrasound data with electronic scanning performed along the second direction, and may preferably be further configured to enable aberration correction along both the first and second directions. This probe configuration may reduce the total number of transducer elements required while still allowing for directional scanning and correction of beam distortion, thereby facilitating efficient and cost-effective implementation of the ultrasound stimulation system.

### BRIEF DESCRIPTION OF THE DRAWINGS

Figure 1 shows a training system 10 for generating a domain-specific large vision model DSLVM 14 using volume anatomical data 11 and generating simulated/synthetic ultrasound data 13d.
Figure 2 shows a system 20 for guided ultrasound stimulation based on a trained DSLVM.
Figure 3 shows a volume-mode embodiment of the system 30 with a matrix probe 31b and real-time guidance outputs 37a, 37b, 37c.
Figure 4 shows a 1.75D ultrasonic probe 40 comprising an array 41 of transducer elements 44.
Figure 5 shows a slice-mode embodiment of the system 50 incorporating a pre-procedure scan 53a, orientation sensor 51c, and visual guidance outputs 54a-54d.

### DETAILED DESCRIPTION OF THE DRAWINGS

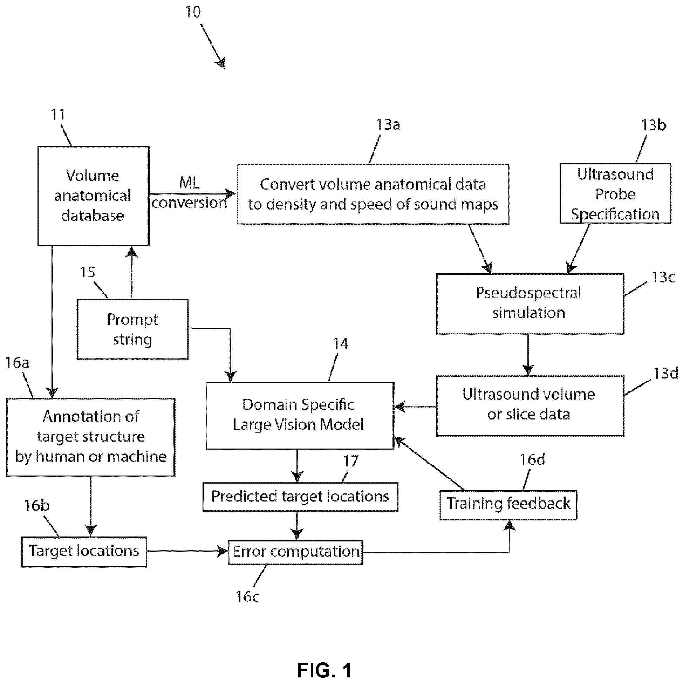

Figure 1 illustrates a training system 10 applying the computer-implemented method for training a domain-specific large vision model (DSLVM) 14 for use in guiding an ultrasound stimulation system to target specific anatomical locations.

The training system 10 comprises a volumetric anatomical database 11 containing volumetric anatomical data representative of one or more subjects. The volumetric anatomical data may include volumetric anatomical scans (e.g. images based on magnetic resonance imaging, MRI, and/or stack(s) of two-dimensional image slices) of target locations representing anatomical structures, which may preferably correspond to cranial or cerebral regions. These scans are used as a necessary input for subsequently generating synthetic ultrasound training data (e.g. in combination with other data as detailed below).

In the illustrated embodiment, the volumetric anatomical scans are processed to produce acoustic property maps 13a, wherein this is an optional feature of the invention. These maps represent spatial distributions of acoustic parameters, such as tissue density or speed of sound (e.g. propagation), and may be derived from the voxel intensity values of the anatomical scans. The volumetric anatomical data (e.g. in a processed form in which they are converted into the acoustic property maps 13a), along with a probe specification 13b defining the functional and hardware characteristics of an ultrasonic probe, are used as inputs for conducting a simulation 13c. The probe specification 13b may define, for example, at least one of: the transducer array geometry, centre frequency, transducer specifications and/or probe orientation relative to the anatomical volume.

The simulation 13c (which is exemplary represented by a module, may be integrated as a function of one or more computing devices executing the computer-implemented method) is configured to generate synthetic ultrasound training data 13d by modelling an ultrasound field produced by the specified ultrasonic probe at multiple positions relative to the anatomical structures (which may be interpreted as representing potential target anatomical locations) represented in the scans. The simulation 13c may use numerical techniques such as pseudo spectral methods, finite-element models, or finite-difference methods.

The synthetic ultrasound training data 13d is used to train the DSLVM 14. It should be noted that the synthetic ultrasound training data 13d may have different formats depending on the specific specification of the ultrasound probe 13b (for example, synthetic ultrasound training data 13d may be configured as synthetic ultrasound volume data for an ultrasound probe specified as configured to acquire volume data, or may be configured as ultrasound slice data for an ultrasound probe specified as configured to acquire slice data). A prompt string 15 may optionally be included to condition the training process toward identifying a particular target anatomical structure. The prompt string may correspond to a user-specified command indicating a target structure (e.g. as the posterior cingulate cortex).

The DSLVM 14 is subsequently trained by processing the synthetic ultrasound training data 13d to generate model parameters (not shown) that, when used by the DSLVM 14, enable the DSLVM 14 to identify target anatomical locations 16b from real-time ultrasound data measured by the specified ultrasonic probe during operation of the ultrasound stimulation system.

As shown in Fig. 1, an optional feature enables the DSLVM 14 to process the synthetic ultrasound data 13d and output predicted anatomical target locations 17. These predictions are compared with reference target anatomical locations 16b, which may optionally be derived from annotations or labels 16a in the original volumetric anatomical data of the volume anatomical database 11. These annotations 16a may correspond to human annotations (e.g. supervised machine learning) or to annotations automatically generated by a machine (e.g. by an unsupervised machine learning method). The result of the comparison is a training error 16c (also referred to as error computation), which is then used to compute (e.g. to generate a) training feedback 16d. The DSLVM 14 is iteratively updated (e.g. by iteratively adjusting the model parameters) based on the training feedback 16d to improve its ability to identify target anatomical locations from ultrasound data.

The trained model parameters resulting from the process are configured to enable the DSLVM 14 to identify target anatomical locations from real-time ultrasound data acquired by the specified ultrasonic probe during actual use of the ultrasound stimulation system (this is also referred to as inference operational mode of the DSLVM 14).

To better illustrate the computer-implemented method of Figure 1, the following non-limiting example is provided: consider a volume-mode implementation in which the volumetric anatomical database 11 contains five anonymized cranial MRI or CT volumes having an isotropic voxel dimension of 1 mm. Preferably, each MRI or CT is automatically converted into an acoustic property map 13a by assigning representative speed-of-sound values (e.g. 1400-1600 m/s for soft tissue, e.g. 2800-4000 m/s for bone). The probe specification 13b may relate to an ultrasonic probe comprising, for example, a 32 × 32 element matrix array (element pitch 0.3 mm; center frequency 800 kHz) capable of single-shot or multiple-firing volume acquisition. During the simulation step 13c the probe may be placed at multiple (e.g. 10 to 50 positions) candidate positions around every head model, and, for each position, a finite-difference time-domain solver generates a synthetic back-scatter volume of 200 × 200 × 120 voxels. These data sets constitute the synthetic ultrasound training data. Optionally, a prompt string 15 (e.g. the text "amygdala") is supplied together with each synthetic volume so that the DSLVM 14 learns to focus on that anatomical structure. For every input, the DSLVM 14 produces a three-dimensional probability map whose voxels indicate the likelihood that they lie inside the amygdala; the highest-probability region is converted into predicted target locations 17. Reference amygdala masks, obtained by segmenting the MRI volumes, serve as ground truth. The predicted target locations 17 are compared with target locations 16b (e.g. derived from annotations of target structures linked to the volumetric anatomical database 11; e.g. provided on the basis of the MRI or CT volumes), to calculate a training error 16c (error computation). This error 16c is back-propagated as training feedback 16d to update the model parameters. Training continues until the training error 16c plateaus for three successive training loops (e.g. epochs), at which point the learned parameters allow the DSLVM 14 to output reliable predicted target anatomical locations 16b from real-time volume ultrasound data captured with the specified matrix probe.

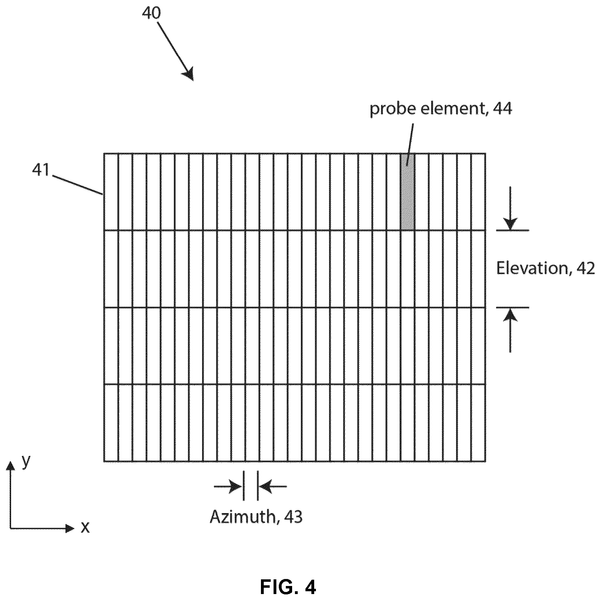

The same workflow can be applied when the ultrasonic probe is configured to acquire slice ultrasound data instead of volume data. In that case the probe specification 13b is replaced by the geometry of a 1-D or 1.75-D array (e.g. the array 41 of Figure 4); the simulation step 13c produces two-dimensional synthetic slices rather than three-dimensional volumes; and the DSLVM 14 is implemented with a 2-D convolutional backbone that processes sequential slices. Apart from these adaptations to probe geometry and network input shape, all other steps-generation of acoustic property maps 13a, use of prompt strings 15, calculation of training errors 16c and feedback 16d-remain unchanged, ensuring that the method disclosed in Fig. 1 is equally applicable to both volume and slice data acquisition modes.

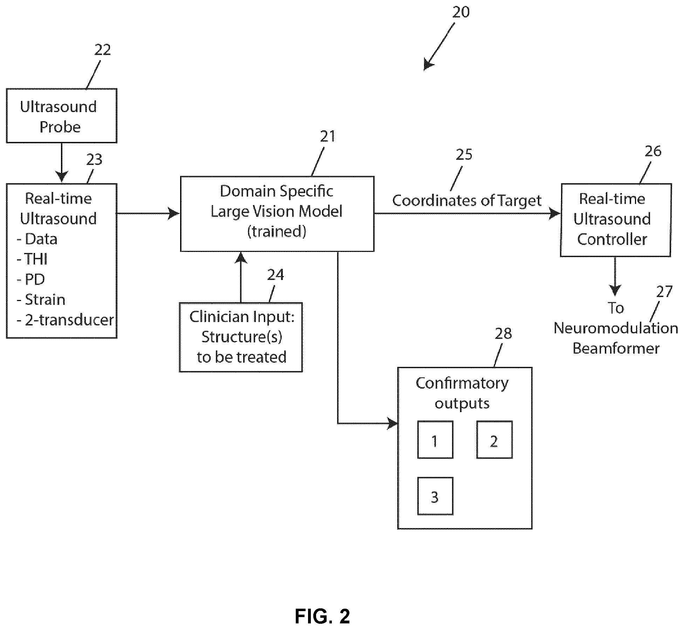

Figure 2 illustrates a system 20 for guiding an ultrasound stimulation comprising a domain-specific large vision model (DSLVM) 14, which may be a DSLVM 21 trained as described in Fig. 1 (e.g. for operating in an inference mode). The system 20 may also be referred to as a system for guided ultrasound stimulation. The system comprises an ultrasonic probe 22, an input interface 24, a computing device comprising the DSLVM 14, a controller 26, and a neuromodulation beamformer 27.

The ultrasonic probe 22 (also referred to as ultrasound probe 22) is configured to acquire real-time ultrasound data 23 from a region of interest within a subject. The region of interest may preferably include cranial and/or cerebral anatomical structures, as already described in the context of the invention. The acquired ultrasound data is used by the system to determine where to deliver focused ultrasound stimulation.

The term "real-time ultrasound data 23" refers to ultrasound imaging data acquired by the ultrasonic probe 22 during system operation, which is processed and interpreted by the DSLVM 21. The real time ultrasound data may include one or more ultrasound imaging modalities, such as tissue harmonic imaging (THI), power Doppler (PD), strain imaging, and/or other imaging signals that represent anatomical, physiological, or functional information related to the region of interest. The real-time ultrasound data 23 may be two-dimensional, slice-based, or volumetric, depending on the capabilities of the ultrasonic probe 22 (which may be configured as a 1D ultrasonic probe, as a 2D ultrasonic probe or as a 1.75D ultrasonic probe), and may be acquired and processed continuously during the targeted stimulation procedure. In some embodiments, the system may be configured to operate in a dual-aperture mode ("2-transducer" in Fig. 2), wherein ultrasound transmission and reception are performed by two distinct transducer arrays. This configuration may allow for improved field-of-view coverage or signal-to-noise optimization in certain anatomical regions.

The input interface 24 is configured to receive a user-specified command indicating a target anatomical structure to be stimulated. This user-specified command may be provided, for example, as a textual input, or as a set of target points which may be preferably marked on a pre-procedure auxiliary volumetric anatomical scan. These input modes are preferably supported by the system, either separately or in combination.

The term "pre-procedure auxiliary volumetric anatomical scan" refers to anatomical imaging data acquired prior to the ultrasound stimulation procedure, such as a magnetic resonance imaging (MRI) scan, computed tomography (CT) scan, or any other modality capable of producing a three-dimensional anatomical reference representation. The scan is considered "auxiliary" in that it is not acquired by the system 20 in real time, but instead serves as a static anatomical reference to support localisation, targeting, and spatial registration. The scan may be used, for example, to define the target anatomical structure/location, to provide input for annotations, or to assist in aligning the real-time ultrasound data with anatomical locations/landmarks.

The computing device comprises the trained DSLVM 21, which is configured to receive both the real-time ultrasound data 23 and the user-specified command via the input interface 24. Based on these inputs, the DSLVM 21 generates anatomical target coordinates 25 corresponding to the target anatomical location, which corresponds to the target anatomical structure indicated with the user-specified command.

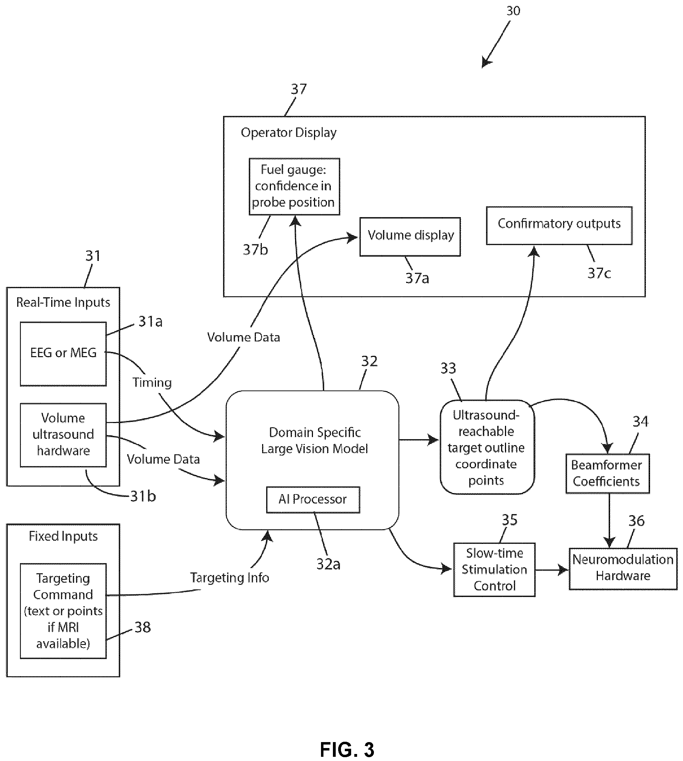

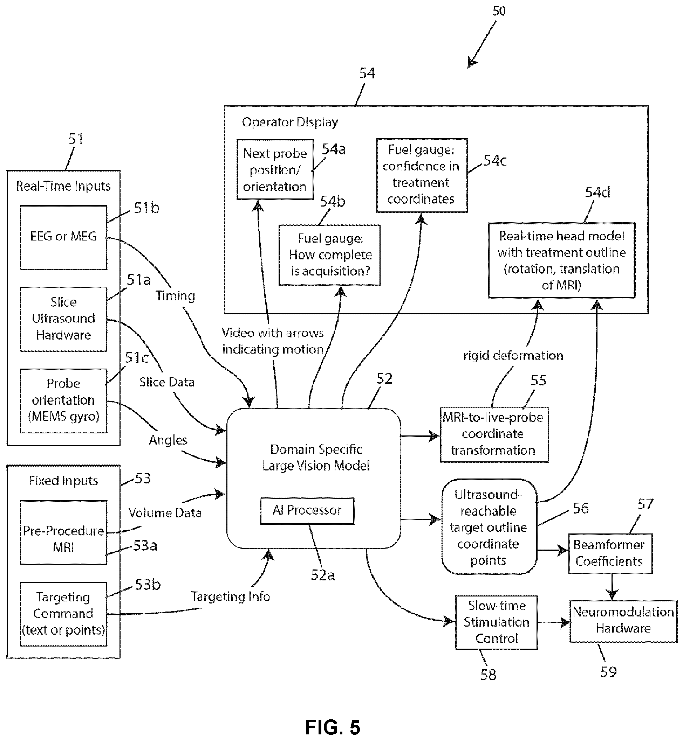

In some embodiments, the system 20 may further comprise an optical sensor (e.g. one or more cameras) or a LIDAR unit configured to acquire additional spatial information about the subject, such as head shape, facial contours, or external anatomical landmarks. This data may be provided to the DSLVM 21 to assist in aligning the real-time ultrasound data (e.g. with a pre-procedure scan, if available), or to improve spatial registration of the ultrasonic probe 22 with the subject's anatomy. The spatial data may be processed together with the real-time ultrasound data 23 by the DSLVM 21 or by a separate registration module. This feature is also broadly compatible with the embodiments shown in Figures 3 and 5.

The controller 26 (also referred to as real-time ultrasound controller 26) is configured to compute beamforming parameters based on the anatomical target coordinates 25 determined by the DSLVM 21. These parameters are then used by the neuromodulation beamformer 27, which is operatively coupled to the ultrasonic probe 22. In some embodiments, the neuromodulation beamformer 27 may be rigidly attached to (or integrated into) the ultrasonic probe 22. The neuromodulation beamformer 27 is configured to generate and deliver focused ultrasound stimulation to the target anatomical location defined by the anatomical target coordinates 25.

Optionally, the DSLVM 21 may be further configured to generate one or more confirmatory outputs 28 to assist an operator (e.g. a clinician) in reviewing or validating the computed anatomical target coordinates 25. The confirmatory outputs 28 may include, for example, a graphical display of a treatment region or a visual representation of the anatomical structures identified. These confirmatory outputs may be shown on an output interface associated with the system. The provision of such confirmatory outputs is an optional advantageous feature of the system and may not be present in all embodiments.

Figure 3 illustrates a system 30 for guiding an ultrasound stimulation procedure using a matrix ultrasonic probe 31b (also referred to as volume ultrasound hardware or 2D ultrasonic probe) configured to acquire volume ultrasound data from a region of interest within a subject. The system 30 is compatible with the system 20 described in Figure 2 and may be regarded as a specific embodiment that comprises additional features on the basis of the system shown in Figure 2. These features may include, for example, the use of a matrix probe 31b for volumetric acquisition, physiological sensors 31a for real-time feedback, and a more advanced output interface 37 (also referred to as operator display) for providing confirmatory visual outputs 37c and additional information. The region of interest may preferably include cranial and/or cerebral anatomical structures, as previously described.

The system 30 comprises a computing device 32a (exemplary represented in Fig. 3 as an "Al processor") including a domain-specific large vision model (DSLVM) 32, a real-time ultrasound controller 34 (represented in Fig. 3 with the label "Beamformer Coefficients" 34), a neuromodulation beamformer 36 (represented in Fig. 3 with the label "Neuromodulation Hardware" 36), and an output interface 38. The DSLVM 32 may be trained using the method and system described in connection with Figure 1.

The ultrasonic probe 31b is configured to acquire real-time volume ultrasound data from the region of interest. The term "real-time ultrasound data" (represented in Fig. 3 with the label "Volume Data") as used throughout the description includes volumetric data of the type acquired by the probe 31b. The DSLVM 32 is configured to receive the real-time volume ultrasound data and to generate anatomical target coordinates 33 corresponding to the target anatomical location indicated by a user-specified command received via the input interface 38.

The real-time ultrasound controller 34 is configured to compute beamforming parameters based on the anatomical target coordinates 33 generated by the DSLVM 32. The neuromodulation beamformer 36 is operatively coupled to the ultrasonic probe 31b (in some embodiments, the neuromodulation beamformer 36 may be rigidly attached to -or integrated into- the ultrasonic probe 31B) and is configured to deliver focused ultrasound stimulation to the target anatomical location based on the beamforming parameters/coefficients computed by the controller 34.

The system 30 may optionally and advantageously comprise one or more physiological sensors 31a configured to monitor a physiological activity of the subject during ultrasound stimulation. For example, when the system is intended to be used for stimulation of head areas, the physiological sensors 31a may advantageously comprise electroencephalographic (EEG) or magnetoencephalographic (MEG) sensors. The DSLVM 32 may be further configured to receive the measured physiological activity in real time and to generate timing control signals 35 based on the measured physiological activity. The neuromodulation beamformer 36 may be further configured to use the timing control signals 35 to synchronise the delivery of ultrasound stimulation with a physiological state of the subject.

The output interface 37 of the system 30 of Figure 3 is configured to provide confirmatory outputs 37c to the operator (e.g. a clinician). In some embodiments, the output interface 37 may include a display 37c showing the treatment region corresponding to the anatomical target coordinates 25. The output interface 37 may also be configured to provide a real-time visualisation 37a (also referred to as "Volume Display" 37a) of the acquired volume ultrasound data (i.e. acquired via the ultrasonic probe 31b), which may be used to monitor anatomical context for targeting purposes. The acquired volume ultrasound data may be directly provided to the real-time visualisation 37a (e.g. without having to be processed by the DSLVM 32). Optionally, the output interface 37 may also be configured to display a graphical indicator 37b representing a confidence level determined by the DSLVM 32. In the illustrated embodiment of Fig. 3, the graphical indicator 37b is also labelled as a "fuel gauge" and may indicate the suitability of the current spatial location of the ultrasonic probe 31b for delivering ultrasound stimulation to the target anatomical location (e.g. the one selected via the input interface 38).

Figure 4 illustrates an embodiment of a 1.75D ultrasonic probe 40, which is generally compatible with the ultrasound stimulation system 20 described in Figure 2 and is particularly suitable for integration into the slice-mode embodiment of the system 50 depicted in Figure 5. The ultrasonic probe 40 is configured for acquiring slice ultrasound data and comprises an array 41 of transducer elements 44.

The array 41 extends along a first direction "y" and a second direction "x", which are mutually orthogonal. Each transducer element 44 has a first dimension 42 extending along the first direction "y" and a second dimension 43 extending along the second direction "x". The first dimension 42 is larger than the second dimension 43. The first direction "y" may also be referred to as an elevation direction, and the second direction "x" may also be referred to as an azimuth direction.

The array 41 is configured to acquire slice ultrasound data with electronic scanning performed along the second direction x. In this configuration, directional steering is enabled along the azimuth direction (i.e. direction x), while the greater extent of the transducer elements along the elevation direction (i.e. direction y) enhances signal sensitivity and beam focusing. Preferably, the array 41 may also be configured to enable aberration correction in both the first direction "y" and the second direction "x", even though active beam steering is performed only along the second direction "x". This configuration may improve image quality and targeting accuracy while maintaining a reduced number of transducer elements compared to full two-dimensional matrix arrays.

The ultrasound probe 40 configuration shown in Figure 4 is well suited for systems where slice-based acquisition is sufficient for anatomical targeting. In the illustrated embodiment, the array 41 comprises a plurality of parallel columns of transducer elements 44 arranged along the second direction "x". Each column comprises a number of transducer elements 44 arranged along the first direction "y". The 1.75D ultrasonic probe 40 shown in Figure 4 is divided into a set of narrow, mutually independent columns, each column containing several vertically stacked transducer elements 44 whose height in elevation exceeds their width in azimuth. Thus, the 1.75D ultrasonic probe comprises several narrow columns of elements laid out side-by-side in the azimuth ("x") direction, while each individual element remains comparatively tall in the orthogonal elevation ("y") direction. Electronic steering is available only in azimuth; however, the extra rows let the beam be apodised or gently refocused in elevation, so lateral resolution and slice thickness are markedly better than with a purely 1D array. Stated differently, a 1.75D array offers most of the elevation-focusing benefits of a full 2D matrix ultrasonic probe but with far fewer channels, cabling and power demands, making it attractive for portable or head-mounted brain applications.

Preferably, the number of parallel columns (along direction "x") is greater than the number of transducer elements 44 per column (along direction "y"). In some embodiments, the number of parallel columns may be in the range of 2 to 8 times, preferably 3 to 7 times, and more preferably 3.5 to 7 times, the number of transducer elements 44 per column.

Figure 4 shows a preferred configuration in which each column comprises four transducer elements 44, and the array comprises 27 parallel columns (i.e. the number of parallel columns is 6.75 times the number of transducer elements 44 per parallel column). This configuration enables electronic scanning in the azimuth direction x while reducing the total number of transducer elements compared to a full 2D matrix array, thereby facilitating simplified driving electronics and reduced channel count. Probe 40 may be fabricated using standard bulk piezoelectric materials and conventional array assembly techniques, contributing to cost-effective manufacturing. This type of probe 40 may be particularly advantageous when used in combination with DSLVM-guided targeting in the slice-mode system 50 described in Figure 5.

In the context of the present description, the term "slice-mode system" refers to an embodiment of the ultrasound stimulation system in which the ultrasonic probe is configured to acquire slice ultrasound data (i.e. two-dimensional images) rather than volume ultrasound data. The slice-mode system typically operates by performing directional scanning along a single axis-preferably the azimuth direction-to acquire sequential ultrasound slices through a region of interest. This is in contrast to volume-mode systems (such as the system of Figure 3), which acquire three-dimensional ultrasound data. The slice-mode system 50 is particularly suited for implementation with 1.75D probes as described in Figure 4.

Figure 5 illustrates a slice-mode system 50 for guiding an ultrasound stimulation procedure based on real-time acquisition of slice ultrasound data. System 50 represents an alternative embodiment to the volume-mode system 30 described in Figure 3. Although structurally distinct, system 50 may also comprise one or more components described in connection with Figure 3, as further clarified below.

The system 50 comprises an ultrasonic probe 51a (represented as slice ultrasound hardware 51a in Fig. 5, and which may optionally be configured as the ultrasonic probe 40 described in Figure 4 or as a 1D ultrasonic probe), a computing device 52a (exemplarily represented as an "Al processor" 52a) including a trained domain-specific large vision model (DSLVM) 52, a real-time ultrasound controller 57 (represented in Fig. 5 with the label "Beamformer Coefficients" 57), a neuromodulation beamformer 59 (labelled "Neuromodulation Hardware" 59), and an output interface 54. The system further comprises an input interface 53, which is configured to receive user-specified inputs and auxiliary anatomical information.

The input interface 53 may functionally correspond to the input interface 38 described in Figure 3. The interface 53 is configured to receive a user-specified command indicating a target anatomical structure to be stimulated. This command may be provided as a textual input (e.g. as a prompt) or as a set of target points marked on a pre-procedure auxiliary volumetric anatomical scan 53a, which is also shown as a separate block in Figure 5. The scan 53a provides a three-dimensional anatomical reference (e.g. of the subject to be stimulated) for use during targeting and spatial registration.

The DSLVM 52 may be configured to receive the real-time slice ultrasound data acquired by the ultrasonic probe 51a and the pre-procedure scan 53a via the input interface 53. Based on these inputs, the DSLVM 52 generates anatomical target coordinates 56 corresponding to the target anatomical location indicated by the user-specified command.

The DSLVM 52 may optionally be configured to compare the slice ultrasound data with the pre-procedure scan 53a and to generate various guidance outputs to assist the operator during the targeting process. These outputs may include one or more of:
- A positioning output 54a, which indicates a recommended spatial location for the ultrasonic probe 51a to complete data acquisition from the region of interest;
- A completeness output 54b, which represents a degree of completeness of anatomical coverage provided by the acquired slice ultrasound data; and/or
- A confidence level output 54c (as the one described for Figure 3), which indicates the suitability of the current spatial location of the ultrasonic probe 51a for delivering ultrasound stimulation to the target anatomical location.

Regarding completeness output 54b, the DSLVM 52 may be configured to evaluate a degree of completeness of the slice ultrasound data acquired by the ultrasonic probe 51a from the region of interest. This evaluation may be performed by comparing the acquired slice ultrasound data with the pre-procedure auxiliary volumetric anatomical scan 53a. Based on this comparison, the DSLVM 52 may generate the completeness output 54b, which is representative of the degree of completeness of the acquired data. The DSLVM 52 may also be configured to determine a recommended position of the ultrasonic probe 51a for completing the acquisition of slice ultrasound data, by comparing a current spatial position of the ultrasonic probe 51a (determined based on the real-time ultrasound data acquired by the ultrasound probe 51a, preferably in combination with orientation data provided by the optional probe orientation sensor 51c) with the pre-procedure scan. The positioning output 54a may be generated indicating the next recommended probe position for increasing completeness of the slice ultrasound data of a region of interest of the subject. These outputs may assist the operator in navigating the probe to ensure adequate coverage of the region of interest and data quality for accurate targeting.

The DSLVM 52 may additionally be configured to generate a real-time anatomical model 54d, based on a coordinate transformation 55 of the pre-procedure scan 53a, the slice ultrasound data acquired by the ultrasonic probe 51a, and the user-specified command. The real-time anatomical model 54d may be overlaid with the anatomical target coordinates 56 and displayed on the output interface 54 to assist the operator in visually validating the targeting.

The output interface 54 may be configured to display any or all of the outputs 54a to 54d. In this respect, the graphical confidence output 54c may be presented using a visual format such as a fuel gauge, as previously described for graphical indicator 37b in Figure 3.

The system 50 may further comprise one or more physiological sensors 31a, similar to those described in Figure 3. These sensors may be configured to monitor a physiological activity of the subject during the ultrasound stimulation procedure. In embodiments targeting cranial regions, the sensors 31a may include electroencephalographic (EEG) or magnetoencephalographic (MEG) sensors. The DSLVM 52 may optionally be configured to generate timing control signals 58 based on the measured physiological activity. These signals may be used by the neuromodulation beamformer 59 to synchronize stimulation with the subject's physiological state.

Figure 5, shows an optional configuration in which the system 50 further comprises a probe orientation sensor 51c configured to determine an orientation of the ultrasonic probe. The probe orientation sensor may be configured as a MEMS gyro (Micro-Electro-Mechanical Systems gyroscope) or as an accelerometer, preferably attached to the ultrasonic probe. The DSLVM 52 may be further configured to receive orientation data from the probe orientation sensor 51c for use in determining the target anatomical coordinates 56 and /or for improving precision of the positioning output 54a indicating the next recommended probe position for increasing completeness of the slice ultrasound data of a region of interest of the subject.

The real-time ultrasound controller 57 is configured to compute beamforming parameters based on the anatomical target coordinates 56 determined by the DSLVM 52. These parameters are provided to the neuromodulation beamformer 59, which is operatively coupled to the ultrasonic probe 51a and configured to deliver focused ultrasound stimulation to the anatomical target location based on the computed parameters. The controller 57 and beamformer 59 in Figure 5 correspond functionally to the controller 34 and beamformer 36, respectively, as previously described in the context of Figure 3.

## Claims

1. A computer-implemented method for training with a computer device a domain-specific large vision model (14), DSLVM (14), for guiding an ultrasound stimulation system (20, 30, 50) to target specific anatomical locations, the method comprising:
providing volumetric anatomical data (11), the volumetric anatomical data (11) comprising volumetric anatomical scans of target anatomical locations (16b) representing anatomical structures, which preferably correspond to cranial and/or cerebral anatomical structures;
providing a specification (13b) of an ultrasonic probe (22, 31b, 40, 51a) of the ultrasound stimulation system (20, 30, 50), the specification (13b) comprising a set of technical parameters defining a functional and hardware configuration of an ultrasonic probe (22, 31b, 40, 51a);
generating synthetic ultrasound training data (13d) by simulating (13c) an ultrasound field produced by the specified ultrasonic probe (22, 31b, 40, 51a) at multiple probe positions relative to anatomical structures represented by at least one of the volumetric anatomical scans;
training the DSLVM (14) by inputting the synthetic ultrasound training data (13d) into the DSLVM (14); and
generating, as a result of the training, model parameters that, when used by the DSLVM (14), enable the DSLVM (14) to identify target anatomical locations (16b) from real-time ultrasound data (23) measured by the specified ultrasonic probe (22, 31b, 40, 51a) during operation of the ultrasound stimulation system (20, 30, 50).

2. The method of claim 1, wherein training the DSLVM (14) by inputting the synthetic ultrasound training data (13d) into the DSLVM (14) comprises applying a training loop comprising:
generating predicted target anatomical locations (17) from the DSLVM (14) based on the synthetic ultrasound training data (13d);
comparing the predicted target anatomical locations (17) with reference target anatomical locations (16b) derived from the volumetric anatomical data (11); and
iteratively adjusting the model parameters of the DSLVM (14) by providing training feedback (16d) to the DSLVM (14) based on training errors (16c) computed from the comparison.

3. The method of claim 2, wherein the reference target anatomical locations (16b) are derived from annotations or labels (16a) of target anatomical structures in the volumetric anatomical data (11), the labels (16a) being used during the training loop to compute training errors (16c) for adjusting the model parameters of the DSLVM (14).

4. The method of any of the preceding claims, further comprising selectively providing a prompt string (15) indicating a specific target anatomical structure, such that the synthetic ultrasound training data (13d) is generated for the specific target anatomical structure indicated by the prompt string (15), the prompt string (15) being used to condition the DSLVM (14) during training to associate ultrasound data with target anatomical locations (16b) corresponding to the target anatomical structure indicated in the prompt string (15).

5. The method of any of the preceding claims, wherein generating the synthetic ultrasound training data (13d) comprises generating acoustic property maps (13a) by processing at least one of the volumetric anatomical scans from the volumetric anatomical data (11), the acoustic property maps (13a) representing spatial distributions of acoustic parameters of anatomical structures and being used as input to the simulation (13c).

6. The method of any of the preceding claims, wherein simulating the ultrasound field to generate the synthetic ultrasound training data (13d) comprises modelling ultrasound propagation and scattering using a simulation model comprising one of a pseudo spectral model, a finite-element model, or a finite-difference model.

7. A system (20, 30, 50) for guiding an ultrasound stimulation procedure, the system (20, 30, 50) comprising:
an ultrasonic probe (22, 31b, 40, 51a) configured to acquire real-time ultrasound data (23) from a region of interest within a subject related to a target anatomical location, preferably including cranial and/or cerebral anatomical structures;
an input interface (24, 38, 53) configured to receive a user-specified command indicating a target anatomical location to be targeted, wherein preferably the input interface (24, 38, 53) is configured to receive the user-specified command configured as a textual input and/or as a set of target points, which are preferably marked on a pre-procedure auxiliary volumetric anatomical scan (38, 53a, 53b);
a computing device (32a, 52a) comprising a trained domain-specific large vision model, DSLVM (14), the DSLVM (14) being configured to receive the real-time ultrasound data (23) and the user-specified command (24), and to output anatomical target coordinates (25) of the indicated target anatomical location based on the real-time ultrasound data (23) and the user-specified command (24), wherein the DSLVM (14) is preferably a DSLVM (14) trained with the computer-implemented method of any of claims 1 to 6;
a controller (26) configured to compute beamforming parameters (34, 57) based on the anatomical target coordinates (25, 33, 56); and
a neuromodulation beamformer (27, 36, 59) coupled to the ultrasonic probe (22, 31b, 40, 51a) and configured to generate and deliver focused ultrasound stimulation to target anatomical locations based on the beamforming parameters (34, 57).

8. The system (20, 30, 50) of claim 7, wherein the DSLVM (14) is further configured to generate confirmatory outputs (28) comprising a visual representation of anatomical structures and a treatment region corresponding to the anatomical target coordinates (25), the system (20, 30, 50) further comprising an output interface (37, 54) for providing the confirmatory outputs (28) to an operator through to facilitate verification of targeting prior to or during delivery of ultrasound stimulation;
wherein preferably the output interface (37, 54) is further configured to display a graphical indicator (37b, 54c) representing a confidence level determined by the DSLVM (14) based on user-specified command and on the real-time ultrasound data (23) acquired by the ultrasonic probe (22, 31b, 40, 51a), the confidence level indicating the suitability of the current spatial location of the ultrasonic probe (22, 31b, 40, 51a) for delivering ultrasound stimulation to the location of the target anatomical location specified via user-specified command provided through the input interface (24, 38, 53).

9. The system (30, 50) of claim 8, wherein
the system further comprises one or more physiological sensors (31a) configured to monitor a physiological activity of the subject during ultrasound stimulation at the target location in real time, the physiological sensors (31a) preferably comprising electroencephalographic (EEG) or magnetoencephalographic (MEG) sensors;
the DSLVM (14) is further configured to receive the physiological activity measured by the one or more physiological sensors (31a), and to generate timing control signals (35, 58) based on the physiological activity measured by the one or more physiological sensors (31a); and
the neuromodulation beamformer (27, 36, 59) is further configured to use the timing control signals (35, 58) to synchronise the delivery of ultrasound stimulation with the measured physiological activity of the subject.

10. The system (30) of claim 8 or 9, wherein:
the ultrasonic probe (31b) comprises a matrix array of transducer elements configured to acquire volume ultrasound data from the region of interest within the subject; and
the output interface (37) is further configured to display (37a) a real-time visualisation of the volume ultrasound data acquired by the ultrasonic probe (31b).

11. The system (50) of claim 8, wherein:
the ultrasonic probe (40, 51a) comprises transducer elements (44) configured to acquire slice ultrasound data from the region of interest within the subject, wherein preferably the transducer elements (44) of the ultrasonic probe are arranged forming a matrix array (41) of transducer elements (44); and
the system (50) further comprises a probe orientation sensor (51c) configured to determine an orientation of the ultrasonic probe (40), wherein the DSLVM (14) is further configured to receive orientation data from the probe orientation sensor (51c) for use in determining anatomical target coordinates.

12. The system (50) of claim 11, wherein the DSLVM (14) is further configured to receive, via the input interface (53), a pre-procedure auxiliary volumetric anatomical scan (53a) of the subject as input, the pre-procedure auxiliary volumetric anatomical scan (53a) being configured to provide subject-specific anatomical information for use by the DSLVM (14) in determining anatomical target coordinates (25) from the slice ultrasound data acquired by the ultrasonic probe (40, 51a).

13. The system (50) of claim 12, wherein the DSLVM (14) is further configured to:
evaluate a degree of completeness of the slice ultrasound data acquired by the ultrasonic probe (40, 51a) from the region of interest by comparing the acquired slice ultrasound data with the pre-procedure auxiliary volumetric anatomical scan (53a), and to generate a completeness output (54b) representative of said degree of completeness; and
determine a recommended position of the ultrasonic probe (40) for completing the acquisition of slice ultrasound data from the region of interest by comparing the current spatial position of the ultrasonic probe (40, 51a), determined based on the acquired slice ultrasound data and the pre-procedure auxiliary volumetric anatomical scan (53a), to generate a positioning output (54a) indicating a next recommended probe position for completing the acquisition of slice ultrasound data from the region of interest.

14. The system (50) of claim 12 or 13, wherein the DSLVM (14) is further configured to generate a real-time anatomical model (54d) based on a coordinate transformation of the pre-procedure auxiliary volumetric anatomical scan (53a), the real-time ultrasound data (23) acquired by the ultrasonic probe (40, 51a) and the user-specified command indicating a target anatomical location to be targeted, the real-time anatomical model (54d) being preferably overlaid with the anatomical target coordinates (25, 56) corresponding to the target anatomical location.

15. The system (50) of any of claims 11 to 14, wherein the array (41) of transducer elements (44) of the ultrasonic probe (40) extends along a first direction (y) and a second direction (x) orthogonal to the first direction (y), each transducer element (44) having a first dimension (42) along the first direction (y) and a second dimension (43) along the second direction (x), wherein the first dimension (42) is greater than the second dimension (43), and wherein the array (41) is configured to acquire slice ultrasound data by performing directional scanning along the second direction (x),
wherein preferably the array (41) comprises a plurality of parallel columns of transducer elements (44) arranged along the second direction (x), each column comprising a plurality of transducer elements (44) arranged along the first direction (y), and the number of parallel columns is greater than the number of transducer elements (44) per column,
wherein more preferably, the number of parallel columns is in the range of 2 to 8 times, preferably 3 to 7 times, and more preferably 3.5 to 7 times, the number of transducer elements (44) per column.
